# EUROPÄISCHE PATENTSCHRIFT

(11) **EP 0 611 571 B1**
(45) Veröffentlichungstag und Bekanntmachung des Hinweises auf die Patenterteilung: **19.04.2000**
(21) Anmeldenummer: 94102332.7
(22) Anmeldetag: 16.02.1994
(51) Int. Cl.: A61K 38/00, A61K 9/14, A61K 9/70, A61K 47/42

(54) **Teicoplaninenthaltendes, lokal applizierbares Arzneimittel mit verzögerter Wirkstofffreisetzung**
Teicoplanin containing topical compositions with delayed release of the active agent
Compositions à utilisation topique à base de teicoplanine et à libération contrôlée de l'agent actif

(30) Priorität: 16.02.1993 DE 4304716
(43) Veröffentlichungstag der Anmeldung: 24.08.1994
(73) Patentinhaber: Melzer, Wolfgang Dr., D-80539 München (DE)
(72) Erfinder:
(74) Vertreter: HOFFMANN - EITLE

(56) Entgegenhaltungen:
- EP-A- 0 326 873
- EP-A- 0 460 448
- WO-A-91/13595

## Beschreibung

Bei der Behandlung von umschriebenen Infektionsherden, insbesondere von Knocheneiterungen oder Abszessen im Weichteil, ist eine radikale Herdsanierung durch den Chirurgen notwendig. Zur Abtötung der noch verbleibenden Keime hat sich eine adjuvante, lokale Antibiotika- bzw. Antiseptikatherapie bewährt. Primär werden gemäß dem Standardverfahren der septischen Chirurgie, nach Ausräumung der Infektherde, Antibiotika- bzw. Antiseptikalösungen der Arzneistoffe mittels der sogenannten Saug-Spüldrainage nahe des Infektionsherdes appliziert oder die Arzneistoffe in Kombination mit nicht resorbierbaren Arzneistoffträgersystemen bzw. mit resorbierbaren Arzneistoffträgersystemen eingebracht. Die Nachteile der verschiedenen Techniken sind bekannt und diese Verfahren werden in Ermangelung anderer Systeme und/oder Techniken trotzdem routinemäßig angewendet. Bei den nicht resorbierbaren Arzneistoffträgersystemen, insbesondere den sogenannten Polymethacrylatmaterialien, ist die Entfernung des eingebrachten Trägersystems durch den Chirurgen notwendig. Die Risiken einer erneuten Operation sind bekannt. Weiter ist bekannt, daß die eingebrachten Arzneistoffe im Körper nicht vollständig aus dem Kunststoff freigesetzt werden. Bei resorbierbaren Tragersystemen auf der Basis der sogenannten Fibrinklebeplombe (Kombination des Fibrinklebesystems-Fibrinogen und Thrombin mit einem Arzneistoff) sowie Kollagen, wie z.B. dem Verbund aus Kollagen und Gentamicin, muß das im Körper eingebrachte Material nicht mehr vom Chirurgen entfernt werden. Der Träger wird im Organismus unter vollständiger Freisetzung des Arzneistoffes resorbiert. Von Nachteil ist, insbesondere für das Weichteil, die rasche Freisetzung des eingebrachten Arzneistoffes aus den bioresorbierbaren Trägersystemen.

WO-A-91 13595 offenbart beispielsweise lokal zu applitierendes Teicoplanin, wobei eine verzöperte Freisetzung des Antibiotikums durch die Verkapselung in eine Polymermatrix erfolgt.

"Ophtholmika, Pharmakologie, Biopharmazie und Gelenik der Augenarzneimittel, Hrgg. v.D. Dolder, Stuttgart, Wiss.- Verl.- Ges., 1990, Seite 497" re Feriert über Depot-Augenoirtheimittel auf Basis von Polymer-Matrices, z.b. Fibrin oder Collagen.

Aufgabe der Erfindung ist es, für die lokale Anwendung ein Arzneimittel mit verzögerter Freisetzung zur Verfügung zu stellen.

Gegenstand der Erfindung ist ein lokal applizierbares Arzneimittel mit verzögerter Freisetzung in Form eines Trockenproduktes oder einer Suspension davon in einem inerten flüssigen Träger, enthaltend als schwer wasserlösliches Produkt die Kombination aus A) Teicoplanin mit B) mindestens einem weiteren Arzneimittelwirkstoff mit basischem Charakter.

Teicoplanin ist ein Glykopeptid mit einer sehr komplexen chemischen Struktur. Es enthält Fettsäure-Seitenketten, die die Liphophilie dieses Antibiotikums erhöhen und die gesteigerte Liphophilie dient der ausgezeichneten Penetration in Zellen, Gewebe (auch Knochen und Knorpel). Überraschenderweise wurde gefunden, daß Teicoplanin mit anderen Arzneimittelwirkstoffen mit basischem Charakter schwer wasserlösliche Produkte ergeben, aus denen die Wirktstoffe bei lokaler Applikation verzögert freigesetzt werden.

Die Arzneimittelwirkstoffe mit basischem Charakter enthalten in der Regel basische Gruppen und es wird vermutet, daß sich diese an die Fettsäure-Seitenketten des Teicoplanins anlagern und dann die schwer wasserlöslichen Produkte bilden. Teicoplanin selbst ist gut wasserlöslich. Während Teicoplanin in Wasser gelöst in der Praxis i.V. oder i. M. appliziert wird, sind die Kombinationen aus Teicoplanin mit mindestens einem weiteren Arzneimittelwirkstoff mit basischem Charakter schwer in Wasser löslich und liegen als Trockenprodukt oder als Suspension davon in einem flüssigen Träger, wie Wasser, vor. Dieses Trockenprodukt oder eine Suspension davon, wird direkt auf den Infektionsherd bzw. das infektionsgefährdete Gebiet aufgetragen. Infolge der Schwerlöslichkeit in Wasser und damit auch in der Gewebeflüssigkeit erfolgt die Freisetzung verzögert, so daß der Wirkstoffspiegel über einen längeren Zeitraum erhalten bleibt.

Teicoplanin ist ein gegen gram-positive Erreger wirksames Antibiotikum. Erfindungsgemäß wird es mit einem weiteren Arzneimittelwirkstoff, mit basischem Charakter, kombiniert. Solche Arzneimittelwirkstoffe sind beispielsweise Therapiepräparate auf der Basis von Proteinen wie Immunoglobulinen, wie z.B. der Klassen IgG oder IgM, die zur Infekttherapie Verwendung finden. Hier sind weiter zu nennen, Antibiotika mit in der Regel basischem Charakter, wie Aminoglykoside (Gentamicin, Netilmicin, Tobramicin). Ebenfalls können Zytostatika mit Teicoplanin kombiniert werden.

In Kombination mit Arzneimittelwirkstoffen auf der Basis von Proteinen wie z.B. Immunoglobulinen, dem Fibrinklebesystem oder Kollagen, sind Trägersysteme herstellbar. Mit beispielsweise Kollagen stellt die Kombination ein Trägersystem dar, das mit weiteren Wirkstoffen beladen werden kann, und zwar sowohl mit Wirkstoffen mit wiederum basischem Charakter, die dann aufgrund der Ausbildung eines schwerlöslichen Produktes mit Teicoplanin verzögert freigegeben werden, als auch mit beliebigen anderen, auch nicht basischen Wirkstoffen, die auf dem Trägersystem aufgebracht sind und ohne verzögerte Freigabe direkt abgegeben werden.

Die Menge an Teicoplanin in der erfindungsgemäß zu verwendenden Kombination richtet sich nach dem gewünschten Therapieeffekt und hängt selbstverständlich auch von der Molekülstruktur des weiteren basischen Arzneimittelwirkstoffs ab. Das Verhältnis der Komponenten A) Teicoplanin zu B), dem weiteren Arzneimittelwirkstoff oder den weiteren Arzneimittelwirkstoffen mit basischem Charakter kann in einem weiten Bereich variieren. Die Menge des Teicoplanins soll ausreichen, mit dem weiteren Wirkstoff unter Ausbildung des schwer wasserlöslichen Produktes das Kombinationsprodukt mit verzögerter Freisetzung zu ergeben. Liegt für die Kombination mit der Komponente B) keine ausreichende Menge an Teicoplanin vor, so wird nur ein Teil der Komponente B) unter Ausbildung des schwer wasserlöslichen Produktes festgehalten und dann später verzögert freigegeben, während der nicht an Teicoplanin gebundene Teil sofort wirksam werden kann.

In der Praxis beträgt der Bereich, in Gewichtsteilen ausgedrückt, zwischen A) Teicoplanin und B) einem weiteren Arzneimittelwirkstoff mit basischem Charakter wie Gentamicin 1 : 8 bis 8 : 1, vorzugsweise 4 : 1.

### Beispiele:

### Beispiel 1

Teicoplanin liegt als Handelsprodukt Targocid^{R} in Form einer Trockensubstanz vor. Es werden 12 mg (bzw. 9, 6 oder 3 mg) Teicoplanin mit 2 mg Gentamicin in ein Lyophilisationsglas mit Septum eingewogen. Nach einem partiellen Anlösen mit 1 ml sterilem Aqua dest. wird tiefgefroren und in bekannter Weise lyophilisiert. Man erhält eine pulverförmige Trockensubstanz, die abgewogen und in Ampullen eingebracht wird.

### Beispiel 2

Man arbeitet wie im Beispiel 1, das Anlösen einer Komponente erfolgt jedoch mit einem ml DMSO.

### Beispiel 3

Man arbeitet wie im Beispiel 1, das Anlösen erfolgt jedoch mit 0,5 ml DMSO und 0,5 ml Aqua dest.

### Beispiel 4

Im Handel erhältliche Kollagenschwämme werden mit Teicoplanin wie folgt beschichtet:
ein Kollagenschwamm der Größe 1 cm² in einem entsprechend formschlüssigem Lyophilisationsglas mit Septum wird mit 12 mg (bzw. 9, 6 oder 3 mg) Teicoplanin, gelöst in 0.1 ml DMSO beschickt. Anschließend wird tiefgefroren und in bekannter Weise lyophilisiert.

### Beispiel 5

Das Verfahren gemäß Beispiel 4 wird wiederholt, jedoch wird anstelle des Kollagenschwamms ein mit Gentamicin beschicktes Kollagen (Sulmicin Implant^{R}) verwendet.

## Patentansprüche

1. Lokal applizierbares Arzneimittel mit verzögerter Freisetzung in Form eines Trockenproduktes oder einer Suspension davon in einem inerten flüssigen Träger, enthaltend als schwer wasserlösliches Produkt die Kombination aus
A) Teicoplanin mit
B) mindestens einem weiteren Arzneimittelwirkstoff mit basischem Charakter.

2. Arzneimittel gemäß Anspruch 1, dadurch gekennzeichnet, daß es in Gewichtseinheiten die Komponenten A) zu B) im Verhältnis von 1 : 8 bis 8 : 1 enthält.

3. Arzneimittel gemäß einem der vorhergehenden Ansprüche, dadurch gekennzeichnet, daß die Komponente B) ein Antibiotikum ist.

4. Arzneimittel gemäß Anspruch 3, dadurch gekennzeichnet, daß das Antibiotikum ein Aminoglykosid ist.

5. Arzneimittel gemäß Anspruch 4, dadurch gekennzeichnet, daß das Aminoglykosid Gentamicin, Netilmicin oder Tobramicin ist.

6. Arzneimittel gemäß einem der Ansprüche 1 bis 2, dadurch gekennzeichnet, daß die Komponente B) ein Therapiepräparat auf der Basis von Proteinen ist.

7. Arzneimittel gemäß Anspruch 6, dadurch gekennzeichnet, daß das Protein Kollagen oder ein Immunoglobulin ist, oder dem Fibrinklebesystem angehört.

8. Arzneimittel gemäß Anspruch 7, dadurch gekennzeichnet, daß die Komponente B) Kollagen in Form eines Schwammes oder eines Vlieses ist.

9. Arzneimittel gemäß Anspruch 8, dadurch gekennzeichnet, daß in dem Trägersystem aus Teicoplanin mit Kollagen in Form eines Schwammes oder Vlieses weitere Wirkstoffe eingebracht sind.

10. Verwendung eines Trockenproduktes oder einer Suspension davon in einem inerten flüssigen Träger, enthaltend als schwer wasserlösliches Produkt die Kombination aus
A) Teicoplanin mit
B) mindestens einem weiteren Arzneimittelwirkstoff mit basischem Charakter
zur Herstellung eines lokal applizierbaren Arzneimittels mit verzögerter Freisetzung.

## Claims

1. Locally administrable medicament with delayed release in the form of a dry product or a suspension thereof in an inert liquid excipient, containing the combination of
A) teicoplanin with
B) at least one further medicament active ingredient having basic character as a product which is difficult to dissolve in water.

2. Medicament according to claim 1, characterised in that it contains components A) to B) in the ratio from 1:8 to 8:1 in weight units.

3. Medicament according to one of the preceding claims, characterised in that component B) is an antibiotic.

4. Medicament according to claim 3, characterised in that the antibiotic is an aminoglycoside.

5. Medicament according to claim 4, characterised in that the aminoglcoside is gentamicin, netilmicin or tobramycin.

6. Medicament according to one of claims 1 to 2, characterised in that component B) is a protein based therapeutic preparation.

7. Medicament according to claim 6, characterised in that the protein is collagen or an immunoglobulin, or belongs to the fibrin adhesive system.

8. Medicament according to claim 7, characterised in that component B) is collagen in the form of a sponge or a nonwoven.

9. Medicament according to claim 8, characterised in that further active ingredients are introduced in the excipient system of teicoplanin with collagen in the form of a sponge or nonwoven.

10. Use of a dry product or a suspension thereof in an inert liquid excipient, containing the combination of
A) teicoplanin with
B) at least one further medicament active ingredient having basic character
as a product which is difficult to dissolve in water to produce a locally administrable medicament with delayed release.

## Revendications

1. Composition médicamenteuse à utilisation topique à libération retardée, se présentant sous la forme d'un produit sec ou d'une suspension de celui-ci dans un support liquide inerte, contenant, à titre de produit difficilement soluble dans l'eau, la combinaison constituée de :
A) teicoplanine, avec
B) au moins un autre principe actif médicamenteux ayant un caractère basique.

2. Composition médicamenteuse selon la revendication 1, caractérisée en ce qu'elle contient, en unités de poids, les composants A) et B) en un rapport de 1 : 8 à 8 : 1.

3. Composition médicamenteuse selon l'une des revendications précédente, caractérisée en ce que le composant B) est un antibiotique.

4. Composition médicamenteuse selon la revendication 3, caractérisée en ce que l'antibiotique est un aminoglycoside.

5. Composition médicamenteuse selon la revendication 4, caractérisée en ce que l'aminoglycoside est la gentamicine, la nétilmicine ou la tobramicine.

6. Composition médicamenteuse selon l'une des revendications 1 à 2, caractérisée en ce que le composant B) est une préparation thérapeutique à base de protéines.

7. Composition médicamenteuse selon la revendication 6, caractérisée en ce que la protéine est constituée de collagène ou d'une immunoglobuline, où bien appartient au système agglutinatif à fibrine.

8. Composition médicamenteuse selon la revendication 7, caractérisée en ce que le composant B) est du collagène sous la forme d'une éponge ou d'un matelas de fibres.

9. Composition médicamenteuse selon la revendication 8, caractérisée en ce que, dans le système support à base de la teicoplanine avec du collagène, sous la forme d'une éponge ou d'un matelas de fibres, sont introduits d'autres principes actifs.

10. Utilisation d'un produit sec ou d'une suspension de celui-ci dans un support liquide inerte contenant, à titre de produit difficilement soluble dans l'eau, la combinaison constituée de :
A) teicoplanine, avec
B) au moins un autre principe actif médicamenteux ayant un caractère basique.
pour la préparation d'une composition médicamenteuse à utilisation topique, avec dégagement retardé.
